# EUROPEAN PATENT SPECIFICATION

(11) **EP 3 391 875 B1**
(45) Date of publication and mention of the grant of the patent: **03.11.2021**
(21) Application number: 16876080.9
(22) Date of filing: 16.12.2016
(51) Int. Cl.: A61K 9/51, A61K 31/7088, A61K 9/19, A61K 9/14

(54) **METHOD FOR PREPARING POLYMERIC MICELLE CONTAINING ANIONIC DRUG**
VERFAHREN ZUR HERSTELLUNG VON POLYMEREN MIZELLEN MIT EINEM ANIONISCHEN ARZNEIMITTEL
PROCÉDÉ DE PRÉPARATION DE MICELLE POLYMÈRE CONTENANT UN MÉDICAMENT ANIONIQUE

(30) Priority: 18.12.2015 KR 20150182265
(43) Date of publication of application: 24.10.2018
(73) Proprietor: Samyang Holdings Corporation, Seoul 03129 (KR)
(72) Inventor: NAM, Hye Yeong, Cheongju-si Chungcheongbuk-do 28608 (KR); KIM, Sang-Hee, Daejeon 34022 (KR); SEO, Min-Hyo, Daejeon 35245 (KR); SON, Ji-Yeon, Daejeon 34023 (KR)
(74) Representative: Schrell, Andreas
(86) International application number: PCT/KR2016/014821
(87) International publication number: WO 2017/105138

(56) References cited:
- WO-A1-2017/048018
- KR-A- 20020 022 868
- KR-A- 20100 076 905
- KR-A- 20110 077 818
- KR-A- 20120 078 661
- KR-A- 20130 112 794
- US-A1- 2009 280 181
- US-A1- 2011 268 772
- US-A1- 2013 266 641
- US-A1- 2015 328 152

## Description

### [Technical Field]

The present disclosure relates to a pharmaceutical composition for delivering an anionic drug comprising an anionic drug, and a preparation method thereof as defined in the claims.

### [Background Art]

Many diseases occur as the expression of disease-related genes is increased due to several factors or abnormal activity is exhibited by mutation. siRNA (short interfering RNA) inhibits the expression of a specific gene in a sequence-specific manner at the post-transcription stage, and thus has gained much attention as a gene therapeutic agent. In particular, due to its high activity and precise gene selectivity, siRNA is expected as a nucleic acid therapeutic agent that can resolve the problems of existing antisense nucleotide, ribozyme or the like. siRNA is a short double-stranded RNA and cleaves the mRNA of a gene having a nucleotide sequence complementary thereto to inhibit the expression of the target gene (McManus and Sharp, Nature Rev. Genet. 3:737 (2002); Elbashir, et al., Genes Dev. 15:188 (2001). However, despite these advantages, it is known that not only siRNA is rapidly degraded by nucleases in the blood and rapidly excreted out of the body through the kidney, but also it does not easily pass through a cell membrane because it is strongly negatively charged.

Safe and efficient drug delivery technologies have been studied for a long time and various delivery systems and delivery technologies have been developed, in the field of treatment using an anionic drug, e.g. nucleic acid including siRNA. The delivery systems are largely classified into a viral delivery system using adenovius or retrovirus, etc., and a non-viral delivery system using cationic lipid, cationic polymer, etc.

It is known that the viral delivery systems are exposed to risks, including nonspecific immune responses, and that their commercial use presents a number of problems due to the production processes being complex. Therefore, a recent research trend is to overcome the shortcomings of viral delivery systems using non-viral delivery system. Such non-viral delivery systems are less efficient than viral delivery system, but have the advantages of being accompanied by fewer side effects in terms of the in vivo safety and having a low production cost in terms of economy.

Most representative examples of non-viral delivery systems include cationic lipid-nucleic acid complex (lipoplex) and polycationic polymer-nucleic acid complex (polyplex) using cationic lipid. Many studies have been conducted on the point that these cationic lipids or polycationic polymers form a complex through an electrostatic interaction with an anionic drug, thereby stabilizing an anionic drug and increasing intracellular delivery. However, when using an amount required to obtain sufficient effects, it showed a result that, although it is less than the viral delivery system, it induces serious toxicity so that its use as a medicine is inappropriate. Therefore, there is a need to develop an anionic drug delivery technology that can reduce toxicity by minimizing the amount of cationic polymer or cationic lipid capable of inducing toxicity, and also can be stable in blood and body fluid and enables intracellular delivery so as to obtain sufficient effects.

The document US 2009/280181 A1 discloses in the example 4 a method for preparing a composition for delivering an anionic drug (DNA), comprising: (a) dissolving an anionic drug and a cationic compound (polyethyleneimine, PEI) in an aqueous solvent (water) respectively and mixing them [0109]-[0113]. The mixture obtained in step (a) is lyophilized (with addition of PEG) and further suspended in chloroform [0115]-[0117]. (b) amphiphilic block copolymers (PEG-PBT and GA-PEG-CL-GALA) are added to the product obtained in step (a). A solution is obtained after shaking for 30 min at 37°C [0118].

The document US 2015/328152 A1 discloses in the examples 17-21 the preparation of micelles comprising siRNA, dioTETA and mPEG-PLA-tocopherol by emulsion or double emulsion method. DioTETA (cationic lipid) is dissolved in chloroform.

On the other hand, various attempts have been made to provide a drug delivery system which can solubilize poorly water-soluble drug in the form of polymeric micelle and stabilize them in an aqueous solution by using amphiphilic block copolymer (Korean Patent No. 08180334). However, although these amphiphilic block copolymer can solubilize a poorly-water soluble drug having hydrophobicity by forming polymeric micelles having hydrophobicity therein, negatively charged hydrophilic drugs such as nucleic acids cannot be entrapped in the micelle structure of the polymer and thus, it is not suitable for the delivery of anionic drug including these nucleic acids. Accordingly, the present inventors have disclosed a composition for delivering anionic drugs and various preparation methods thereof which form a complex by electrostatic interactions with nucleic acids and allow the complex to be entrapped in micelle structure of an amphiphilic block copolymer. However, there is still a need for improvement in the production yields of the composition for delivering anionic drugs and methods for preparing formulations for enhancing the stability of nucleic acids.

### [DETAILED DESCRIPTION OF THE INVENTION]

### [Technical Problem]

Under these circumstances, the present inventors have conducted extensive studies to develop a preparation method for increasing the production yield of a composition for delivering an anionic drug and for enhancing the stability of the anionic drug. As a result, the inventors have found that, when an anionic drug such as siRNA and a cationic compound are independently dissolved in an aqueous solvent and mixed to form a complex in a monophase system and then are entrapped in a polymeric micelle, the yield of the anionic drugs can be remarkably improved, and the stability of the anionic drugs can be enhanced, thereby completing the present invention.

Therefore, it is one object of the present invention to provide a method for preparing a composition for delivering an anionic drug in which the production yield of the composition containing an anionic drug and the stability of nucleic acids are enhanced, and a composition for delivering anionic drugs prepared therefrom.

### [ADVANTAGEOUS EFFECTS]

The preparation method according to an embodiment of the present invention allows an anionic drug and a cationic lipid as defined in the claim 1 to form a complex in an aqueous phase, thereby effectively forming a nanoparticular complex by electrostatic interaction. Also, the binding force is increased during the process of removing an aqueous solution through freeze-drying, thereby greatly increasing the yield of finally prepared polymeric micelles. In addition, such preparation method is not only environmentally friendly because of using a relatively small amount of organic solvent, but also the production is extremely easy and mass production is easy. Further, the composition for delivering an anionic drug prepared by the preparation method of an embodiment of the present invention can increase the stability of the anionic drug in blood or body fluid when administered into the body, and in particular, it has the advantage of effectively delivering an anionic drug into the cells without going through the reticuloendothelial system.

### [BRIEF DESCRIPTION OF DRAWINGS]

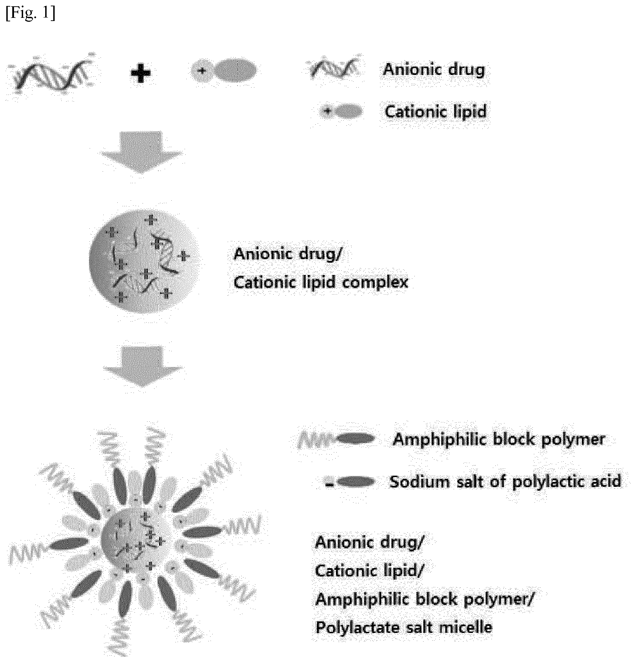

FIG. 1 is a schematic diagram illustrating the structure of a polymeric micelle delivery system prepared by an embodiment of the present invention.

### [DETAILED DESCRIPTION OF THE EMBODIMENTS]

An embodiment of the present invention relates to a method for preparing a composition for an delivering anionic drug which increases the production yield thereof by comprising forming nanoparticles by using electrostatic interaction of an anionic drug and the cationic lipid as defined in claim 1 in an aqueous phase; and incorporating the nanoparticles into polymeric micelles comprising an amphiphilic polymer as defined in claim 1 and optionally a polylactic acid salt to increase the electrostatic interaction and hydrophobic binding, thereby increasing the entrapment efficiency of the anionic drug in the formulation.

Specifically, the composition prepared by an embodiment of the present invention is a composition for delivering anionic drugs having a micelle structure, in which a complex of a drug and a cationic compound is incorporated into the micelle structure of an amphiphilic block copolymer and optionally a polylactic acid salt, and includes an anionic drug, as an active ingredient; the cationic lipid as defined in claim 1; the amphiphilic block copolymer as defined in claim 1; and optionally, a polylactic acid salt, wherein the anionic drug forms a complex by electrostatic interactions with the cationic lipid, and the thus-formed complex is entrapped in the micelle structure formed by the amphiphilic block copolymer and optionally the polylactic acid salt. The preparation method of the present invention is defined in the claims.

Hereinafter, the present invention will be described in more detail. According to the method (i) of the present invention as defined in the claim 1, in step (a), in order to prepare a complex of an anionic drug and the cationic lipid, they are dissolved in an aqueous phase, for example, an aqueous solvent, respectively, and then mixed together. In step (a), the anionic drug and the cationic lipid dissolved in the aqueous solvent form a complex of the anionic drug and the cationic compound in the form of nanoparticles by electrostatic interactions. The aqueous solvent used in the step may be distilled water, injection water, or buffer. The mixing ratio between the aqueous solutions in which the anionic drug and cationic compound are dissolved respectively is not particularly limited, and for example, the volume ratio of the aqueous solution of cationic compound to the aqueous solution of anionic drug may be 1 to 20, more specifically, 1 to 4, but is not limited thereto.

The aqueous solutions are mixed through appropriate mixing means known in the art, and examples of such methods include an ultrasonicator and the like.

The anionic drug used in step (a) is an active ingredient of the composition to be finally prepared, and includes all substances which are negatively charged in the molecule in an aqueous solution and has a pharmacological activity. In a specific embodiment, the anionic property may be provided from at least one functional group selected from the group consisting of a carboxyl group, a phosphate group, and a sulfate group. In addition, in an embodiment of the present invention, the anionic drug may be a peptide, a protein, or a polyanionic drug such as heparin or a nucleic acid.

Further, the nucleic acid may be a nucleic acid drug such as deoxyribonucleic acid, ribonucleic acid or polynucleotide derivatives in which backbone, sugar or base is chemically modified or the terminal thereof is modified, and more specifically, it may be at least one nucleic acid selected from the group consisting of RNA, DNA, siRNA (short interfering RNA), aptamer, antisense ODN (antisense oligodeoxynucleotide), antisense RNA, ribozyme, DNAzyme, and the like. Furthermore, the backbone, sugar or base of the nucleic acid may be chemically modified, or the terminal thereof may be modified for the purpose of increasing blood stability or attenuating an immune response. Specifically, a part of the phosphodiester bond of the nucleic acid may be replaced by a phosphorothioate or boranophosphate bond, or at least one kind of nucleotide in which various functional groups such as methyl group, methoxyethyl group, fluorine, and the like are introduced into 2'-OH position of a part of riboses may be included.

In addition, at least one terminal of the nucleic acid may be modified with at least one selected from the group consisting of cholesterol, tocopherol, and a fatty acid having 10 to 24 carbon atoms. For example, for siRNA, the 5'end, or the 3' end, or both ends of the sense and/or antisense strand may be modified, and preferably, the terminal of the sense strand may be modified.

The cholesterol, tocopherol, and a fatty acid having 10 to 24 carbon atoms include analogs, derivatives, and metabolites of cholesterol, tocopherol, and a fatty acid.

The siRNA may refer to a double-stranded RNA (duplex RNA) which can reduce or suppress the expression of a target gene by mediating the degradation of mRNA complementary to the sequence of the siRNA when present in the same cell as the target gene, or to a single-stranded RNA having a double-stranded region within in the single-stranded RNA. The bond between the double strands is made by a hydrogen bond between nucleotides, all nucleotides in the double strands need not be complementarily bound with each other, and both strands may be separated or may not be separated. According to one embodiment, the length of the siRNA may be about 15 to about 60 nucleotides (it means the number of nucleotides of one of double-stranded RNA, i.e., the number of base pairs, and in the case of a single-stranded RNA, it means the length of double strands in the single-stranded RNA), specifically about 15 to about 30 nucleotides, and more specifically about 19 to about 25 nucleotides.

According to one embodiment, the double-stranded siRNA may have overhang of 1 to 5 nucleotides at 3' or 5' end or both ends. According to another embodiment, it may be blunt without overhang at both ends. Specifically, it may be siRNA disclosed in US Patent Application Publication No. 2002-0086356 and U.S. Patent. No. 7,056,704 .

In addition, the siRNA may have a symmetrical structure with the same lengths of two strands, or it may have a non-symmetrical double-stranded structure with one strand shorter than the other strand. Specifically, it may be a non-symmetrical siRNA (small interfering RNA) molecule of double strands consisting of 19 to 21 nucleotide (nt) antisense; and 15 to 19nt sense having a sequence complementary to the antisense, wherein 5'end of the antisense is blunt end, and the 3'end of the antisense has 1 to 5 nucleotide overhang. Specifically, it may be siRNA disclosed in International Publication WO 2009/078685.

In an embodiment, the anionic drug may be included in an amount of 0.001% to 10% by weight, specifically 0.01% to 5% by weight based on the total weight of the finally prepared composition. If the amount is less than 0.001 % by weight, the amount of delivery system is too large compared to the drug, and thus, side effect may be caused by the delivery system, and if it exceeds 10% by weight, the size of the micelle may become too large so that the stability of the micelle may be decreased and the loss during filter sterilization may be increased.

According to the method (ii) of the present invention as defined in the claim 1, the cationic lipid the cationic compound and the anionic drug forms a complex by electrostatic interactions in an aqueous phase, and the complex is dehydrated through freeze-drying to form a rigid complex of an anionic drug and a cationic compound. Thus, the cationic compound may be a lipid which can form a complex with the anionic drug by electrostatic interactions, and is soluble in aqueous phase.

The cationic lipid according to the invention is defined in claim 1 and is capable of forming a complex by electrostatic interactions with the anionic drug. The cationic lipids described herein but not being a part of the invention are N,N-dioleyl-N,N- dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-diolooyloxy)propylamine (DODMA), N,N,N-tridimethyl-(2,3-dioleoyloxy)propylamine (DOTMA), 1,2-diacyl-3-trimethylammonium-propane (TAP), 1,2-diacyl-3-dimethylammonium-propane (DAP), 3β-[N—(N',N',N'-trimethylaminoethane)carbamoyl]cholesterol (TC-cholesterol), 3β-[N—(N',N'-dimethylaminoethane)carbamoyl]cholesterol (DC-cholesterol), 3β-[N—(N'-monomethylaminoethane)carbamoyl]cholesterol (MC-cholesterol), 3β-[N-(aminoethane)carbamoyl]cholesterol (AC-cholesterol), cholesteryloxypropane-1-amine (COPA), N-(N'-aminoethane)carbamoylpropanoic tocopherol (AC-tocopherol), and N— (N'-methylaminoethane)carbamoylpropanoic tocopherol (MC-tocopherol). When such a cationic lipid is used, it is preferable that polycationic lipid having high cation density in the molecule is used in a small amount in order to decrease toxicity induced by the cationic lipid, and more specifically, the cationic lipid may have one functional group having cationic property in aqueous solution per molecule. Such lipids are 3β-[-N—(N',N',N'-trimethylaminoethane)carbamoyl]cholesterol (TC-cholesterol), 3β[N— (N',N'-dimethylaminoethane)carbamoyl]cholesterol (DC-cholesterol), 3β[N—(N'-monomethylaminoethane)carbamoyl]cholesterol (MC-cholesterol), 3β[N-(aminoethane)carbamoyl]cholesterol (AC-cholesterol), N-(1-(2,3-dioleoyloxy)propyl-N,N,N-trimethylammoniumchloride (DOTAP), N,N-dimethyl-(2,3-dioleoyloxy)propylamine (DODMA), and N,N,N-trimethyl-(2,3-dioleoyloxy)propylamine (DOTMA).

In addition, described are herein but are not a part of the invention lipids having a plurality of functional groups having cationic properties in an aqueous solution per molecule. Such lipids are N,N-dioleyl-N,N-dimethylammoniumchloride (DODAC), N,N-distearyl-N,N-dimethylammoniumbromide (DDAB), 1,2-diacyl-3-trimethylammonium-propane (TAP), and 1,2-diacyl-3-dimethylammonium-propane (DAP).

The cationic lipid according to the invention is the cationic lipid in which an amine functional group of 1 to 12 oligoalkyleneamines is bonded with a saturated or unsaturated hydrocarbon having 11 to 25 carbon atoms, and the cationic lipid may be represented by Chemical Formula 1 below. 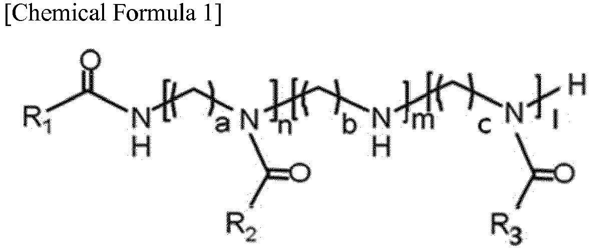 in the formula 1,
n, m and 1 are each 0 to 12, with a proviso that 1 ≤ n + m + 1 ≤ 12, a, b and c are each 1 to 6, R₁, R₂ and R₃ are each independently hydrogen or a saturated and unsaturated hydrocarbon having 11 to 25 carbon atoms, with a proviso that at least one of R₁, R₂ and R₃ is a saturated or unsaturated hydrocarbon having 11 to 25 carbon atoms.

Preferably, n, m and 1 are independently an integer of 0 to 7, wherein 1 ≤ n+m+1 ≤ 7.

Preferably, a, b and c may be from 2 to 4.

Preferably, R₁, R₂ and R₃ are each independently at least one selected from the group consisting of lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, docosahexaenyl, and cerotyl.

Specific examples of the cationic lipid may be at least one selected from the group consisting of monooleoyl triethylenetetramide, dioleoyl triethylenetetramide, trioleoyl triethylenetetramide, tetraoleoyl triethylenetetramide, monolinoleoyl tetraethylene pentaamide, dilinoleoyl tetraethylene pentaamide, trilinoleoyl tetraethylene pentaamide, tetralinoleoyl tetraethylene pentaamide, pentalinoleoyl tetraethylene pentaamide, monomyristoleoyl diethylenetriamide, dimyristoleoyldiethylene triamide, monooleoyl pentaethylenehexamide, dioleoyl pentaethylenehexamide, trioleoyl pentaethylenehexamide, tetraoleoyl pentaethylenehexamide, pentaoleoyl pentaethylenehexamide, and hexaoleoyl pentaethylenehexamide.

Described are herein but are not part of the invention the cationic polymers selected from the group consisting of chitosan, glycol chitosan, protamine, polylysine, polyarginine, polyamidoamine (PAMAM), polyethylenimine, dextran, hyaluronic acid, albumin, polymeric polyethylene imine (PEI), polyamine, and polyvinylamine (PVAm).

The cationic lipid used in the present invention may be included in an amount of 0.01% to 50% by weight, specifically 0.1% to 10% by weight based on the total weight of the finally prepared composition. If the amount of the cationic lipid is less than 0.01% by weight, it may not be sufficient to form a complex with the anionic drug, and if it exceeds 50% by weight, the size of the micelle may become too large so that the stability of the micelle may be decreased and the loss during filter sterilization may be increased.

The cationic lipid binds with the anionic drug by electrostatic interactions in an aqueous phase so as to form a complex.

According to a specific embodiment, the ratio of quantity of electric charge of the cationic compound that is the lipid as defined in claim 1 (N) and the anionic drug (P) (N/P: the ratio of the positive electric charge of the cationic compound to the negative electric charge of the anionic drug) is 0.1 to 128, specifically 0.5 to 64, more specifically 1 to 32, even more specifically 1 to 24, and most specifically 6 to 24. If the ratio (N/P) is less than 0.1, the cationic compound cannot sufficiently bind to the anionic drug, and thus it is advantageous to have the ratio of 0.1 or more so that the cationic compound and the anionic drug can form a complex including a sufficient amount of anionic drugs by electrostatic interaction. In contrast, if the ratio (N/P) exceeds 128, toxicity may be induced, and thus it is preferable to have the ratio of 128 or less.

The step (b) is a step of dissolving an amphiphilic block copolymer as defined in claim 1 and optionally a polylactic acid salt in an aqueous solvent or an organic solvent and mixing the solution with the mixture obtained in step (a).

When the amphiphilic block copolymer and optionally the polylactic acid salt are dissolved in an aqueous solvent and mixed, the preparation of a composition for delivering anionic drugs is carried out in the aqueous phase, in which the complex of the anionic drug-cationic compound in the form of nanoparticles is entrapped in the micelle structure formed by the amphiphilic block copolymer and optionally the polylactic acid salt.

Herein, the aqueous solvent is the same as the aqueous solvent used in step (a).

In addition, the amphiphilic block copolymer is an A-B type block copolymer including a hydrophilic A block and a hydrophobic B block. The A-B type block copolymer forms a core-shell type polymeric micelle in an aqueous solution, wherein the hydrophobic B block forms a core (inner wall) and the hydrophilic A block forms a shell (outer wall).

In this regard, the hydrophilic A block is at least one selected from the group consisting of monomethoxy polyethylene glycol, or polyethylene glycol. The hydrophilic A block may have a number average molecular weight of 200 Dalton to 50,000 Dalton, specifically 1,000 Dalton to 20,000 Dalton, more specifically 1,000 Dalton to 5,000 Dalton.

Further, if necessary, a functional group or a ligand that can reach a specific tissue or cell, or a functional group capable of promoting intracellular delivery may be chemically conjugated to the terminal of the hydrophilic A block so as to control the distribution of the polymeric micelle delivery system formed by the amphiphilic block copolymer and optionally the polylactic acid salt in the body or increase the efficiency of the intracellular delivery of the polymeric micelle delivery system. The functional group or ligand may be at least one selected from the group consisting of monosaccharide, polysaccharide, vitamin, peptide, protein, and an antibody to a cell surface receptor. More specifically, the functional group or ligand may be at least one selected from the group consisting of anisamide, vitamin B9 (folic acid), vitamin B12, vitamin A, galatose, lactose, mannose, hyaluronic acid, RGD peptide, NGR peptide, transferrin, an antibody to a transferrin receptor, and the like.

The hydrophobic B block is a polymer having biocompatibility and biodegradability, and it is polylactide, polyglycolide or copolymer thereof. According to another embodiment, the hydrophobic B block may have a number average molecular weight of 50 Dalton to 50,000 Dalton, specifically 200 Dalton to 20,000 Dalton, more specifically 1,000 Dalton to 5,000 Dalton. In addition, in order to increase hydrophobicity of the hydrophobic block and to thereby improve the stability of the micelle, tocopherol, cholesterol, or a fatty acid having 10 to 24 carbon atoms may be chemically conjugated to a hydroxyl group of the hydrophobic block end.

The amphiphilic block copolymer including the hydrophilic block (A) and the hydrophobic block (B) may be included in an amount of 40% to 99.98% by weight, specifically 85% to 99.8% by weight, more specifically 90% to 99.8% by weight, based on the total dry weight of the composition. If the amount of the amphiphilic block copolymer is less than 40% by weight, the size of the micelle may become too large so that the stability of the micelle may be decreased and the loss during filter sterilization may be increased, and if the amount exceeds 99.98% by weight, the amount of anionic drugs that can be incorporated may become too small.

Further, as for the amphiphilic block copolymer, the composition ratio of the hydrophilic block (A) and the hydrophobic block (B) may be in the range of 40% to 70% by weight, specifically 50% to 60% by weight, based on the weight of the copolymer. If the ratio of the hydrophilic block (A) is less than 40% by weight, it may be difficult to form a micelle because the solubility of the polymer in water is low, and thus, it is preferable that the ratio of the hydrophilic block (A) is 40% by weight or more so that the copolymer has solubility in water sufficient to form micelles. In contrast, if it exceeds 70% by weight, hydrophilicity may be too high so that the stability of the polymeric micelle is lowered, and thus, it is difficult to use it as a solubilizing composition for the anionic drug/cationic compound complex. Therefore, it is preferable that the ratio of the hydrophilic block (A) is 70% by weight or less in consideration of the stability of micelles.

The terminal hydroxyl group of the hydrophobic B block may be modified by at least one selected from the group consisting of cholesterol, tocopherol, and a fatty acid having 10 to 24 carbon atoms.

In addition, the polylactic acid salt (for example, PLANa) may be included in the inner wall of the micelle as a separate component from the amphiphilic block copolymer, and is distributed in the core (inner wall) of the micelle to strengthen the hydrophobicity of the core and to thereby stabilize the micelle, and at the same time, it plays a role of effectively avoiding the reticuloendothelial system (RES) in the body. That is, the carboxylic acid anion of the polylactic acid salt binds to the cationic complex more effectively than the polylactic acid so as to reduce the surface potential of the polymeric micelle, thereby reducing the positive charge of the surface potential compared to a polymeric micelle containing no polylactic acid salt and thus is less trapped by the reticuloendothelial system. Therefore, it has the advantage of having excellent delivery efficiency to a desired site (for example, cancer cells, inflammatory cells, etc.).

The polylactic acid salt preferably has a number average molecular weight of 500 Dalton to 50,000 Dalton, specifically 1,000 Dalton to 10,000 Dalton. If the molecular weight is less than 500 Dalton, the hydrophobicity is too low so that it may be difficult to exist in the core (inner wall) of the micelles, and if the molecular weight exceeds 50,000 Dalton, there is a problem that the particle size of the polymeric micelles becomes large.

The polylactic acid salt may be used in an amount of 1 to 200 parts by weight, specifically 10 to 100 parts by weight, more specifically 30 to 60 parts by weight, based on 100 parts by weight of the amphiphilic block polymer. If the amount of the polylactic acid salt exceeds 200 parts by weight based on 100 parts by weight of the amphiphilic block polymer, the size of the micelle increases and thus, filtration using a sterilized membrane may become difficult, and if the amount is less than 1 part by weight, the desired effects of stabilizing micelles and effectively avoiding the reticuloendothelial system by enhancing hydrophobicity cannot be obtained sufficiently.

According to one embodiment, the amphiphilic block copolymer may be used in an amount of 10 to 1,000 parts by weight and the polylactic acid salt may be used in an amount of 5 to 500 parts by weight based on 1 part by weight of the anionic drug. Preferably, the amphiphilic block copolymer may be used in an amount of 50 to 800 parts by weight, more preferably 100 to 500 parts by weight. Preferably, the polylactic acid salt may be used in an amount of 10 to 300 parts by weight, more preferably 50 to 100 parts by weight.

According to one preferred embodiment, the polylactic acid salt of the present invention may be at least one selected from the group consisting of compounds represented by Chemical Formulae 2 to 7 below:

[Chemical Formula 2] RO-CHZ-[A]ₙ-[B]ₘ-COOM

in the formula 2, A is -COO-CHZ-; B is -COO-CHY-, -COO-CH₂CH₂CH₂CH₂CH₂- or -COO-CH₂CH₂OCH₂; R is a hydrogen atom or an acetyl, benzoyl, decanoyl, palmitoyl, methyl, or ethyl group; Z and Y are each a hydrogen atom or a methyl or phenyl group; M is Na, K, or Li; n is an integer of 1 to 30; and m is an integer of 0 to 20;

[Chemical Formula 3] RO-CHZ-[COO-CHX]ₚ-[COO-CHY' ]_{q}-COO-CHZ-COOM

in the formula 3, X is a methyl group; Y' is a hydrogen atom or a phenyl group; p is an integer of 0 to 25 and q is an integer of 0 to 25, with a proviso that p+q is an integer of 5 to 25; R is a hydrogen atom or an acetyl, benzoyl, decanoyl, palmitoyl, methyl or ethyl group; M is Na, K, or Li; Z is a hydrogen atom, a methyl or phenyl group;

[Chemical Formula 4] RO-PAD-COO-W-M'

in the formula 4, W-M' is 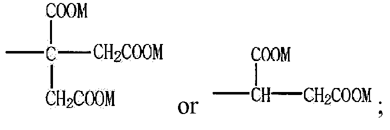 PAD is selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; R is a hydrogen atom, or a acetyl, benzoyl, decanoyl, palmitoyl, methyl or ethyl group; M is independently Na, K, or Li;

[Chemical Formula 5] S-O-PAD-COO-Q

in the formula 5, S is 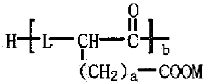 ; L is -NR₁- or -O-, herein R₁ is a hydrogen atom or C₁₋₁₀ alkyl; Q is CH₃, CH₂CH₃, CH₂CH₂CH₃, CH₂CH₂CH₂CH₃, or CH₂C₆H₅; a is an integer of 0 to 4; b is an integer of 1 to 10; M is Na, K, or Li; PAD is at least one selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; 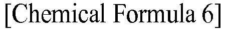 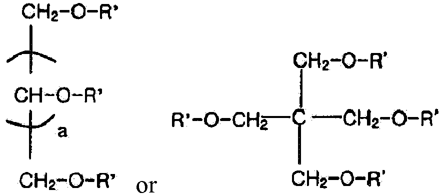 in the formula 6, R' is -PAD-O-C(O)-CH₂CH₂-C(O)-OM, PAD is selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; M is Na, K, or Li; and a is an integer of 1 to 4;

[Chemical Formula 7] YO-[-C(O)-(CHX)ₐ-O-]ₘ-C(O)-R-C(O)-[-O-(CHX' )_{b}-C(O)-]ₙ-OZ

in the formula 7, X and X' are independently hydrogen, alkyl having 1 to 10 carbon atoms, or aryl having 6 to 20 carbon atoms; Y and Z are independently Na, K, or Li; m and n are independently integers of 0 to 95, with a proviso that 5 < m + n < 100; a and b are independently integers of 1 to 6; R is -(CH₂)ₖ-, a divalent alkenyl having 2 to 10 carbon atoms, a divalent aryl having 6 to 20 carbon atoms, or a combination thereof, herein k is an integer of 0 to 10.

The polylactic acid salt is preferably a compound represented by Chemical Formula 2 or Chemical Formula 3.

According to a specific embodiment, the amphiphilic block copolymer allows the complex of the anionic drug and the cationic compound to entrap in the micelle structure in an aqueous solution by forming a micelle wall optionally together with the polylactic acid salt, wherein the ratio of the weight of the complex of the anionic drug and the cationic compound (a) to the weight of the amphiphilic block copolymer (b) [a/b×100; (the weight of the anionic drug + the weight of the cationic compound)/the weight of the amphiphilic block copolymer X 100] may be 0.001% to 100% by weight, specifically 0.01% to 50% by weight, more specifically 0.1 % to 10% by weight. If the weight ratio is less than 0.001% by weight, the amount of the complex of the anionic drug and the cationic lipid may become too low, and thus it may be difficult to satisfy the effective amount with which the anionic drug can effectively function. In contrast, if it exceeds 100% by weight, a micelle structure of appropriate size may not be formed considering the molecular weight of the amphiphilic block copolymer and the amount of the complex of the anionic drug and the cationic compound.

According to one embodiment of the present invention, the preparation method may further include a step (c) of stabilizing the mixture obtained in step (b) at a temperature of 0°C to 50°C for 5 minutes to 60 minutes. The stabilization may be carried out by allowing the mixture to stand still or with stirring. The stabilizing condition may be preferably 0°C to 50°C, more specifically 4°C to 30°C for 5 minutes to 1 hour, more specifically 10 minutes to 30 minutes, but is not limited thereto. If the time is less than 5 minutes, the complex is not stabilized, and if it exceeds 1 hour, precipitation of the complex may be generated.

Meanwhile, according to still another embodiment, the method (ii) as defined in claim 1 for preparing a composition for delivering anionic drugs according to the present invention includes: (a') independently dissolving an anionic drug and the cationic lipid as defined in claim 1 in an aqueous solvent and mixing them, followed by freeze-drying;
(b-1) dissolving the freeze-dried product obtained in step (a') in an organic solvent;
(b-2) mixing the solution obtained in step (b-1) with an aqueous solvent; and
(b-3) removing the organic solvent from the mixture obtained in step (b-2).

Herein, the amphiphilic block copolymer as defined in claim 1 and optionally the polylactic acid salt may be dissolved in the organic solvent of step (b-1) or the aqueous solvent of step (b-2).

In step (a'), the mixture obtained by independently dissolving the anionic drug and the cationic lipid as defined in claim 1 in an aqueous solvent, followed by mixing is freeze-dried. In step (a'), the complex is effectively formed by electrostatic interaction, and the binding strength of the thus-formed complex increases during the process of removing water through freeze-drying.

Furthermore, in step (b-1), the dried nanoparticular complex is dissolved in an organic solvent, and the organic solvent used herein may be at least one selected from the group consisting of acetone, ethanol, methanol, methylene chloride, chloroform, dioxane, dimethyl sulfoxide, acetonitrile, ethyl acetate and acetic acid. Preferably, it may be at least one selected from the group consisting of ethanol, dimethyl sulfoxide, ethyl acetate, and acetic acid.

The organic solvent may include a fusogenic lipid. The fusogenic lipid may be at least one selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

The step (b-2) is a step of entrapping the mixture of the anionic drug-cationic lipid as defined in claim 1 in the form of nanoparticles inside of the micelle structure formed by the amphiphilic block copolymer as defined in claim 1 and optionally the polylactic acid salt by mixing the solution obtained in step (b-1) in an aqueous solvent, wherein the aqueous solvent used may be distilled water, injection water, or buffer. The amount of the aqueous solvent used is not particularly limited and may be, for example, 1 to 10, more specifically 1 to 5 folds, on a volume basis relative to the amount of organic solvent of step (b-1), but is not limited thereto.

In addition, the amphiphilic block copolymer as defined in claim 1 and the polylactic acid salt used in step (b-1) or (b-2) may be the same kind and may be used in the same amount as the amphiphilic block copolymer and the polylactic acid salt mentioned above.

Further, in step (b-3), an aqueous solution of the polymeric micelle is obtained by removing the organic solvent in the mixture prepared in the step (b-2) by evaporation.

Furthermore, according to one preferred embodiment, the preparation method of the present invention may further include a step (d) of carrying out freeze-drying by adding a freeze-drying aid after step (b-3).

According to still another embodiment, the preparation method of the present invention may further include sterilizing the aqueous solution of the polymeric micelle obtained in step (b-3) with a sterilizing filter before the freeze-drying of step (d).

The freeze-drying aid used in an embodiment of the present invention may is added to allow the freeze-dried composition to maintain a cake form or to help uniformly dissolve the amphiphilic block copolymer composition in a short period of time during reconstitution after freeze-drying, and specifically, it may be at least one selected from the group consisting of lactose, mannitol, sorbitol, and sucrose. The amount of the freeze-drying aid may be 1% to 90% by weight, more specifically 10% to 60% by weight, based on the total dry weight of the composition.

According to an embodiment of the preparation method of the present invention, an anionic drug and the cationic lipid as defined in claim 1 are allowed to form a complex in an aqueous phase, thereby effectively forming a nanoparticular complex by electrostatic interaction. Also, the binding force is increased during the process of removing an aqueous solution through freeze-drying, thereby greatly increasing the yield of finally prepared polymeric micelles. Further, the preparation method is not only environmentally friendly because of using a relatively small amount of organic solvent, and also reproducibility is maintained by preventing the composition ratio from changing due to the tendency of the cationic lipid to adhere to the manufacturing apparatus, containers or the like, and the production is extremely easy. Further, mass production can be easily made by converting the anionic drugs into hydrophobic drug particles through the formation of the complex.

In addition, in the composition prepared according to an embodiment of the present invention, since the complex of the anionic drug and the cationic lipid as defined in claim 1 maintains the state of being entrapped inside of the micelle structure formed by the amphiphilic block copolymer as defined in claim 1 and optionally the polylactic acid salt, the stability thereof in blood or body fluid is enhanced.

Further described is herein but is not a part of the invention a composition for delivering anionic drugs including the polymeric micelle prepared by the above-mentioned preparation method.

According to the preparation method of the present invention, the anionic drug binds to the cationic lipid as defined in claim 1 through electrostatic interactions to form the anionic drug-cationic lipid complex, and the polymeric micelle structure in which the complex is entrapped inside of the micelle structure formed by the amphiphilic block polymer as defined in claim 1 and optionally the polylactic acid salt is prepared. The schematic structure of the polymeric micelle delivery system prepared by one embodiment of the present invention is shown in FIG. 1

As shown in FIG. 1, the micelle structure formed by the amphiphilic block polymer and optionally the polylactic acid salt has a structure in which the complex of the anionic drug and the cationic compound is entrapped inside of the formed micelle, wherein the hydrophilic portion of the amphiphilic block copolymer forms the outer wall of the micelle, and the hydrophobic portion of the amphiphilic block copolymer and the polylactic acid salt contained as a separate component from the amphiphilic block copolymer forms the inner wall of the micelle.

The disclosure relating to the anionic drug, cationic compound, amphiphilic block polymer, polylactic acid salt, and the like, which are the constituents of the composition, are the same as those described in the preparation method according to the present invention.

According to a preferred embodiment, the particle size of the micelle in the composition may be 10 to 200 nm, more specifically, 10 to 100 nm. In addition, the standard charge of the micelle particles is -20 to 20 mV, more specifically -10 to 10 mV. The particle size and the standard charge are preferable considering the stability of the micelle structure, the contents of the constitutional ingredients, and absorption and stability of anionic drugs in the body.

The composition containing the anionic drug-cationic lipid complex entrapped in the micelle structure of the amphiphilic block copolymer and optionally the polylactic acid salt according to an embodiment of the present invention may be administered intravenously, intramuscularly, subcutaneously, orally, intra-osseously, transdermally, topically, and the like, and it may be manufactured into various oral or parenteral formulations suitable for the administration routes. Examples of the oral formulations include tablets, capsules, powders, and solutions, and examples of the parenteral formulations include eye drops, injections, and the like. In one preferred embodiment, the formulation may be injection formulation. For example, in case that the composition is freeze dried, it may be prepared in the form of an injection formulation by reconstituting it with distillated water for injection, a 0.9% saline solution, a 5% dextrose aqueous solution, and the like.

### [DETAILED DESCRIPTION OF THE EMBODIMENTS]

Hereinafter, the present invention will be explained in detail by way of Examples. However, these Examples are only to illustrate the invention and the scope of the invention is not limited thereto in any manner.

### [Comparative Example 1] Preparation of siRNA/1,6-dioleoyl triethylenetetramide (dio-TETA)/mPEG-PLA-tocopherol (2k-1.7k) /PLANa (1.7k)-containing composition

126 µg of 1,6 dioTETA was dissolved in 6.3 µℓ of chloroform, and 5 µg of siRNA was dissolved in 4 µℓ of distilled water. 0.5 mg of PLANa (1.7 k) was dissolved in 10 µℓ of chloroform, and 1 mg of mPEG-PLA-tocopherol (2k-1.7k) was dissolved in 20 µℓ of chloroform. 3.7 µℓ of chloroform was added so that the volume ratio of the organic layer to the water layer as a whole was 10 times. 44 µℓ of chloroform was added to 4 µℓ of the solution in which 1 mg of mPEG-PLA-tocopherol had been dissolved in chloroform, which is equivalent to 0.2 mg of mPEG-PLA-tocopherol (20% by weight), and the mixture was added to a 1-necked round flask, and the solvent was removed by distillation under reduced pressure using a rotary evaporator.

The dioTETA solution, PLANa solution and 0.8 mg solution of mPEG-PLA-tocopherol were mixed, and an emulsion was prepared using an ultrasonicator while adding the siRNA aqueous solution dropwise. The emulsion was added to a 1-necked round flask coated with 0.2 mg of mPEG-PLA-tocopherol, and the solvent was removed by distillation under reduced pressure using a rotary evaporator. 100 µℓ of distilled water was added to the flask and gently shaken to dissolve, thereby preparing a siRNA/dioTETA/mPEG-PLA-tocopherol (2k-1.7k)/PLANa-containing composition (Comparative Example 1).

### [Comparative Examples 2-3] Preparation of siRNA/1,6-dioleoyl triethylenetetramide (dio-TETA)/mPEG-PLA-tocopherol (2k-1.7k) /DOPE / (PLANa)-containing compositions

Comparative Examples 2 and 3 were prepared in the same manner as in Comparative Example 1, except that the composition ratios were changed. 5 µg of the siRNA was dissolved in 4 µℓ of distilled water, and the composition excluding the siRNA was dissolved in chloroform so that the ratio of the organic layer to the water layer was 10 times. In the case of Comparative Example 2, 94.5 µg g of 1,6 dioTETA was dissolved in 5 µℓ of chloroform, 1 mg of mPEG-PLA-tocopherol (2k-1.7k) was dissolved in 20 µℓ of chloroform, and 104 µg of DOPE was dissolved in 5.2 µℓ of chloroform, and 9.8 µℓ of chloroform was added. In the case of Comparative Example 3, 94.5 µg g of 1,6 dioTETA was dissolved in 5 µℓ of chloroform, 1 mg of mPEG-PLA-tocopherol (2k-1.7k) was dissolved in 20 µℓ of chloroform, 0.3 mg of PLANa was dissolved in 9.8 µℓ of chloroform, and 104 µg of DOPE was dissolved in 5.2 µℓ of chloroform. 44 µℓ of chloroform was added to 4 µℓ of the solution in which 1 mg of mPEG-PLA-tocopherol had been dissolved in chloroform, which is equivalent to 0.2 mg of mPEG-PLA-tocopherol (20% by weight), and the mixture was added to a 1-necked round flask, and the solvent was removed by distillation under reduced pressure using a rotary evaporator.

The dioTETA solution, 0.8 mg solution of mPEG-PLA-tocopherol, and (or) PLANa solution or DOPE solution were mixed, and an emulsion was prepared using an ultrasonicator while adding the siRNA aqueous solution dropwise. The emulsion was added to a 1-necked round flask coated with 0.2 mg of mPEG-PLA-tocopherol, and the solvent was removed by distillation under reduced pressure using a rotary evaporator. 100 µℓ of distilled water was added to the flask and gently shaken to dissolve, thereby preparing siRNA/dioTETA/mPEG-PLA- tocopherol (2k-1.7k)/DOPE-containing composition (Comparative Example 2), siRNA/dioTETA/mPEG-PLA- tocopherol (2k-1.7k)/PLANa/DOPE-containing composition (Comparative Example 3)

**[Table 1]**

| | Composition | ratio | siRNA | lipid | polyme r 1 | polyme r 2 | fusogenic lipid |
|---|---|---|---|---|---|---|---|
| Comparati ve Example1 | siRNA/dioTETA /mPEG-PLA-tocopherol (2k-1.7k)/PLANa (1.7k) | 5-24-1-0.5 | 5 µg | 150 µg | 1 mg | 0.5 mg | 0 mg |
| Comparati ve Example2 | siRNA/dioTETA /mPEG-PLA-tocopherol (2k-1.7k)/ DOPE | 5-18-1-1 | 5 µg | 94.5 µg | 1 mg | 0 mg | 104.2 mg |
| Comparati ve Example3 | siRNA/dioTETA /mPEG-PLA-tocopherol (2k-1.7k)/PLANa (1.7K)/DOPE | 5-18-1-0.3-1 | 5 µg | 150 µg | 1 mg | 0.3 mg | 104.2 mg |

(Unit of each component in the composition ratio is as follows: siRNA: µg, lipid: N/P ratio, polymer: mg, mole ratio of fusogenic lipid: lipid. Polymer 1 refers to mPEG-PLA-tocopherol: Polymer 2 refers to PLANa. The same applies to the following tables.)

### [Examples 1-2] Preparation of siRNA/1,6-dioTETA/mPEG-PLA-tocopherol (2k-1.7k)/PLANa (1.7k)-containing compositions (preparation of formulations in aqueous phase)

126 µg of 1,6 dioTETA was dissolved in 252 µℓ of distilled water and then placed in an ultrasonic washer for 10 minutes to reduce the particle size. 5 µg of siRNA was dissolved in 4 µℓ of distilled water, and 1 mg of mPEG-PLA-tocopherol (2k-1.7k) and 500 µg of PLANa (1.7k) were dissolved in 10 µℓ and 2 µℓ of distilled water, respectively. siRNA and 1,6-dioTETA were first mixed, and then mPEG-PLA-tocopherol and PLANa were mixed. Distilled water was added so that the volume ratio was 1:1. The mixture of siRNA and 1,6 dioTETA and the mixture of mPEG-PLA-tocopherol and PLANa were mixed dropwise under ultrasonication. After kept at 4°C for 10 minutes for stabilization of the formulation, the mixture was filtered through a 0.45 µm hydrophilic PVDF filter to eliminate large particles. (Example 1)

In Example 2, the composition was prepared with 500 µg of mPEG-PLA-tocopherol (2k-1.7k) and 100 µg of PLANa (1.7k) according to the procedure in Example 1.

**[Table 2]**

| | Composition | ratio | siRNA | lipid | polymer1 | polymer2 |
|---|---|---|---|---|---|---|
| Example 1 | siRNA/dioTET A/m PEG-PLA-tocopherol(2k-1.7k)/PLANa (1.7k) | 5-24-1-0.5 | 5 µg | 126 µg | 1 mg | 0.5 mg |
| Example 2 | siRNA/dioTETA/m PEG-PLA-tocopherol(2k-1.7k)/PLANa (1.7k) | 5-24-0.5-0.1 | 5 µg | 126 µg | 0.5 mg | 0.1 mg |

### [Example 3] Preparation of siRNA/1,6-dioTETA/mPEG-PLA-tocopherol (2k-1.7k)/PLANa (1.7k)-containing composition (preparation method of forming siRNA/dioTETA nanoparticles in aqueous phase and entrapping them into polymeric micelle in emulsion)

5 µg of siRNA was dissolved in 4 µℓ of distilled water, 126 µg of dioTETA was dissolved in 126 µℓ of distilled water, and then mixed dropwise under ultrasonication. The mixture was freeze-dried to a powder state, and the powder was dissolved with a solution in which 300 µg of PLANa was dissolved in 50 µℓ of ethyl acetate. A solution in which 1 mg of mPEG-PLA-tocopherol (2k-1.7k) was dissolved in 100 µℓ of distilled water was added dropwise to the mixture of siRNA, dioTETA and PLANa to prepare an emulsion using an ultrasonicator. The prepared emulsion was placed in a 1-necked round flask and subjected to distillation under reduced pressure using a rotary evaporator to selectively remove ethyl acetate to prepare siRNA/1,6-dioleoyl triethylenetetramide(dioTETA)/mPEG-PLA-tocopherol (2k-1.7k)/PLANA-containing polymeric micelles.

### [Examples 4-6] Preparation of siRNA/1,6-dioTETA/mPEG-PLA-tocopherol (2k-1.7k)/PLANa (1.7k)-containing compositions (preparation method of forming siRNA/dioTETA nanoparticles in aqueous phase and entrapping them into polymeric micelle in emulsion)

siRNA/dioTETA/mPEG-PLA-tocopherol (2k-1.7k) /PLANa (1.7k)-containing compositions were prepared in a manner similar to Example 3, with a proviso that the mixing order of the compositions was changed. The composition, type and amount of the solvent, and the preparation procedure are the same, but the following examples are divided according to which solvent the composition is dissolved in. A complex emulsion was prepared by using distilled water dissolved with PLANa after dissolving siRNA and dioTETA powder in ethyl acetate containing mPEG-PLA-tocopherol (2k-1.7k) ( Example 4). A complex emulsion was prepared by using distilled water dissolved with mPEG-PLA-tocopherol (2k-1.7k) and PLANa, after dissolving the powder in ethyl acetate ( Example 5). In addition, a complex emulsion was prepared by using distilled water after dissolving the powder in ethyl acetate containing mPEG-PLA-tocopherol (2k-1.7k) and PLANa (Example 6).

**[Table 3]**

| | Composition | ratio | siRNA | lipid | polymer1 | polymer2 |
|---|---|---|---|---|---|---|
| Example 3-6 | siRNA/dioTET A/m PEG-PLA-tocopherol(2k-1.7k)/PLANa (1.7k) | 5-24-1-0.3 | 5 µg | 126 µg | 1 mg | 0.3 mg |

### [Examples 7-12] Preparation of siRNA/1,6-dioTETA/mPEG-PLA-tocopherol (2k-1.7k)/PLANa (1.7k)-containing compositions (preparation method of forming siRNA/dioTETA nanoparticles in aqueous phase and entrapping them into polymeric micelle in emulsion)

siRNA/dioTETA/mPEG-PLA-tocopherol (2k-1.7k)/PLANa (1.7k)-containing compositions were prepared in the same manner as Example 3, except that different compositions were used.

The compositions obtained in Examples 7 to 12 are summarized in Table 4 below:

**[Table 4]**

| | Composition | ratio | siRNA | lipid | polymer1 | polymer2 |
|---|---|---|---|---|---|---|
| Example 7 | siRNA/dioTET A /mPEG-PLA-tocopherol(2k-1.7k)/PLANa (1.7k) | 5-24-2-0.3 | 5 µg | 126 µg | 2 mg | 0.3 mg |
| Example 8 | siRNA/dioTET A /mPEG-PLA-tocopherol(2k-1.7k)/PLANa (1.7k) | 5-24-0.5-0.3 | 5 µg | 126 µg | 0.5 mg | 0.3 mg |
| Example 9 | siRNA/dioTET A /mPEG-PLA-tocopherol(2k-1.7k)/PLANa (1.7k) | 5-18-1-0.1 | 5 µg | 95 µg | 1 mg | 0.1 mg |
| Example 10 | siRNA/dioTET A /mPEG-PLA-tocopherol(2k-1.7k)/PLANa (1.7k) | 5-18-0.5-0.1 | 5 µg | 95 µg | 0.5 mg | 0.1 mg |
| Example 11 | siRNA/dioTET A /mPEG-PLA-tocopherol(2k-1.7k)/PLANa (1.7k) | 5-12-1-0.05 | 5 µg | 63 µg | 1 mg | 0.05 mg |
| Example 12 | siRNA/dioTET A /mPEG-PLA-tocopherol(2k-1.7k)/PLANa (1.7k) | 5-12-0.5-0.05 | 5 µg | 63 µg | 0.5 mg | 0.05 mg |

### [Examples 13-14] Preparation of siRNA/1,6-dioTETA/mPEG-PLA-tocopherol (2k-1.7k)/DOPE-containing compositions (preparation method of forming siRNA/dioTETA nanoparticles in aqueous phase and entrapping them into polymeric micelle in emulsion)

siRNA/dioTETA/mPEG-PLA-tocopherol (2k-1.7k)/DOPE-containing compositions were prepared in the same manner as Example 6, except that different compositions were used.

The compositions obtained in Examples 13 and 14 are summarized in Table 5 below:

**[Table 5]**

| | Composition | ratio | siRNA | lipid | polymer1 | fusogenic lipid |
|---|---|---|---|---|---|---|
| Example 13 | siRNA/dioTETA/mPEG-PLA-tocopherol(2k-1.7k)/DOPE | 5-18-1-0.5 | 5 µg | 95 µg | 1 mg | 52 µg |
| Example 14 | siRNA/dioTETA/mPEG-PLA-tocopherol(2k-1.7k)/DOPE | 5-18-1-1 | 5 µg | 95 µg | 1 mg | 104 µg |

### [Examples 15-16] Preparation of siRNA/1,6-dioTETA/mPEG-PLA-tocopherol (2k-1.7k)/PLANa (1.7K)/DOPE-containing compositions (preparation method of forming siRNA/dioTETA nanoparticles in aqueous phase and entrapping them into polymeric micelle in emulsion)

siRNA/dioTETA/mPEG-PLA-tocopherol (2k-1.7k)/PLANa (1.7K)/DOPE-containing compositions were prepared in the same manner as Example 6, except that different compositions were used.

The compositions obtained in Examples 15 and 16 are summarized in Table 6 below:

**[Table 6]**

| | Composition | ratio | siRNA | lipid | polyme r1 | polyme r2 | fusogen ic lipid |
|---|---|---|---|---|---|---|---|
| Example 15 | siRNA/dioTET A/mPE G-PLA-tocopherol(2k-1.7k)/PLANa(1.7k)/DO PE | 5-18-1-0.1_1 | 5 µg | 95 µg | 1 mg | 0.1 mg | 104 µg |
| Example 16 | siRNA/dioTET A/mPE G-PLA-tocopherol(2k-1.7k)/PLANa(1.7k)/DO PE | 5-18-1-0.3_1 | 5 µg | 95 µg | 1 mg | 0.3 mg | 104 µg |

### [Experimental Example 1] Comparison of siRNA contents of polymeric micelles according to preparation method

The siRNA content was weighed to confirm how the yield of nanoparticles varied according to each preparation method and composition.

The amount of siRNA in the prepared polymeric micelles was quantified using the modified Bligh & Dyer extraction method. The polymeric micelles were dissolved in 50 mM sodium phosphate and 75 mM NaCl (pH 7.5) to form a Bligh-Dyer monophase and extracted with 100 mM sodium phosphate, 150 mM NaCl (pH 7.5) and chloroform to quantify the siRNA in the aqueous solution layer with the Ribogreen reagent (Invitrogen).

The siRNA content of the siRNA/dioTETA/mPEG-PLA-tocopherol(2k-1.7k)/PLANa (1.7k) polymeric micelles according to the preparation method of preparing formulations in aqueous phase and forming siRNA/dioTETA nanoparticles in an aqueous phase, followed by entrapping them into polymeric micelles is shown in Table 7 below.

**[Table 7]**

| | Preparation method | ratio | siRNA content (%) |
|---|---|---|---|
| Comparative Example1 | Preparation method using water miscible solvent | 5-24-1-0.5 | 42 |
| Example 1 | Preparation method using aqueous phase | 5-24-1-0.5 | 72 |
| Example 2 | | 5-24-0.5-0.1 | 82 |
| Example 3 | Preparation method using formation of siRNA/dioTETA nanoparticle in aqueous phase, and entrapment of the siRNA/dioTETA nanoparticles into polymer micelle in emulsion | 5-24-1-0.3 | 63 |
| Example 4 | | 5-24-1-0.3 | 61 |
| Example 5 | | 5-24-1-0.3 | 71 |
| Example 6 | | 5-24-1-0.3 | 60 |

The siRNA contents of Examples 7 to 12 having different compositions according to the preparation method of forming siRNA/dioTETA nanoparticles in an aqueous phase and entrapping them into polymeric micelles in the emulsion are shown in Table 8 below.

**[Table 8]**

| | Composition | ratio | siRNA content (%) |
|---|---|---|---|
| Example 7 | siRNA/dioTETA/mPEG-PLA-tocopherol(2k-1.7k)/PLANa (1.7k) | 5-24-2-0.3 | 70 |
| Example 8 | | 5-24-0.5-0.3 | 69 |
| Example 9 | | 5-18-1-0.1 | 50 |
| Example 10 | | 5-18-0.5-0.1 | 54 |
| Example 11 | | 5-12-1-0.02 | 42 |
| Example 12 | | 5-12-0.5-0.02 | 44 |

The siRNA contents of Examples 13 to 16 having different compositions according to the preparation method of forming siRNA/dioTETA nanoparticles in an aqueous phase and entrapping them into polymeric micelles in the emulsion are shown in Table 9 below.

**[Table 9]**

| | Composition | ratio | siRNA content (%) |
|---|---|---|---|
| Comparative Example2 | siRNA/dioTETA/mPEG-PLA-tocopherol(2k-1.7k)/DOPE | 8-18-1-1 | 52 |
| Example 13 | | 5-18-1-0.5 | 62 |
| Example 14 | | 5-18-1-1 | 67 |
| Comparative Example3 | siRNA/dioTETA/mPEG-PLA-tocopherol(2k-1.7k)/PLANa (1.7K)/DOPE | 5-18-1-0.3-1 | 60 |
| Example 15 | | 5-18-1-0.1-1 | 85 |
| Example 16 | | 5-18-1-0.3-1 | 89 |

As shown in Tables 7, 8 and 9, the siRNA contents for the compositions of Examples 1 to 16 prepared according to embodiments of the preparation method of the present invention were remarkably superior to those of Comparative Examples. Such a result demonstrates that the method of forming siRNA/dioTETA nanoparticles in an aqueous phase enhances the efficient interaction between siRNA and the cationic lipids, thereby efficiently entrapping the siRNA in micelles.

### [Comparative Example 4] Preparation of siRNA/1,6-dioTETA/mPEG-PLA-tocopherol (2k-1.7k)-containing composition (Method for preparing complex emulsion)

126 µg of dioTETA was dissolved in chloroform, and 5 µg of siRNA was dissolved in distilled water. 1 mg of mPEG-PLA-tocopherol (2k-1.7k) was dissolved in chloroform. The dioTETA and mPEG-PLA-tocopherol were mixed, and an emulsion was prepared using an ultrasonicator while adding the siRNA dropwise. A complex emulsion was prepared using an ultrasonicator while adding the emulsion to the distilled water. The complex emulsion was placed in a 1-neck round flask, and chloroform was removed by distillation under reduced pressure using a rotary evaporator to prepare a siRNA/dioTETA/mPEG-PLA-tocopherol (2k-1.7k)-containing composition.

**[Table 10]**

| | Composition | ratio | siRNA | lipid | polymer |
|---|---|---|---|---|---|
| Comparative Example4 | siRNA/1,6-dioTETA/mPEG-PLA-tocopherol (2k-1.7k) | 5-24-1 | 5 µg | 126 µg | 1 mg |

### [Example 17] Preparation of siRNA/1,6-dioTETA/mPEG-PLA-tocopherol (2k-1.7k)-containing composition (preparation method of forming siRNA/dioTETA nanoparticles in aqueous phase and entrapping them into polymeric micelle in emulsion)

5 µg of siRNA was dissolved in 4 µℓ of distilled water, 126 µg of dioTETA was dissolved in 126 µℓ of distilled water, and then mixed dropwise under ultrasonication. The mixture was freeze-dried to a powder state, and the powder was dissolved in 50 µℓ of ethyl acetate. A solution in which 1 mg of mPEG-PLA-tocopherol (2k-1.7k) was dissolved in 100 µℓ of distilled water was added dropwise to the mixture of siRNA, dioTETA and PLANa to prepare an emulsion using an ultrasonicator. The prepared emulsion was placed in a 1-necked round flask and subjected to distillation under reduced pressure using a rotary evaporator to selectively remove ethyl acetate to prepare siRNA/1,6-dioleoyl triethylenetetramide(dio-TETA)/mPEG-PLA-tocopherol (2k-1.7k)-containing polymeric micelles.

**[Table 11]**

| | Composition | ratio | siRNA | lipid | polymer1 |
|---|---|---|---|---|---|
| Example 17 | siRNA/dioTETA/mPEG-PLA-tocopherol(2k-1.7k) | 5-24-1 | 5 µg | 126 µg | 1 mg |

### [Experimental Example 2] Comparison of siRNA contents according to the preparation methods of siRNA/1,6-dioTETA/mPEG-PLA-tocopherol (2k-1.7k) polymeric micelles

The siRNA content was quantified to confirm how the yield of nanoparticles varied according to each preparation method.

The amount of siRNA in the prepared siRNA/dioTETA/mPEG-PLA-tocopherol (2k-1.7k)-containing polymeric micelles was quantified using the modified Bligh & Dyer extraction method. The polymeric micelles were dissolved in 50 mM sodium phosphate and 75 mM NaCl (pH 7.5) to form a Bligh-Dyer monophase and extracted with 100 mM sodium phosphate, 150 mM NaCl (pH 7.5) and chloroform to quantify the siRNA in the aqueous solution layer with the Ribogreen reagent (Invitrogen).

The comparison results for the siRNA contents entrapped in the micelles of Comparative Example 4 and Experimental Example 17 are shown in Table 12 below.

**[Table 12]**

| | Preparation method | ratio | siRNA content (%) |
|---|---|---|---|
| Comparative Example4 | Preparation using Complex emulsion | 5-24-1 | 50 |
| Example 17 | Preparation method using formation of siRNA/dioTETA nanoparticles in aqueous phase, and entrapment of the | 5-24-1 | 72 |
| | siRNA/dioTETA nanoparticles into polymer micelle in emulsion | | |

As shown in Table 12, the siRNA content of Example 17 prepared according to an embodiment of the preparation method of the present invention is remarkably superior to that of Comparative Example.

### [Experimental Example 3] Comparison of stability of polymeric micelles (Heparin competition assay)

Heparin competition assay was performed to investigate the *in vitro* stability according to each preparation method and composition. 10 µℓ of the formulation (300 ng of siRNA) was treated with 40 *µ*g of heparin and allowed to react at room temperature for 10 minutes. Then, the dissociated siRNA was measured by electrophoresis. The formulation has higher stability as the siRNA dissociation gets lower, and the comparison of the stability according to the composition ratio is shown in Table 13 below.

**[Table 13]**

| | Preparation method | ratio | siRNA Dissociation(%) |
|---|---|---|---|
| Comparative Example1 | Preparation method using water miscible solvent | 5-24-1-0.5 | 43 |
| Example 1 | Preparation method using water phase | 5-24-1-0.5 | 25 |
| Example 2 | | 5-24-0.5-0.1 | 15 |
| Example 3 | Preparation method using formation of siRNA/dioTETA nanoparticle in water phase, and entrapment of the siRNA/dioTETA nanoparticles | 5-24-1-0.3 | 9 |
| Example 4 | | 5-24-1-0.3 | 9 |
| Example 5 | | 5-24-1-0.3 | 13 |
| Example 6 | | 5-24-1-0.3 | 13 |
| Example 7 | | 5-24-2-0.3 | 27 |
| Example 8 | into polymer micelle in emulsion | 5-24-0.5-0.3 | 2 |
| Example 9 | | 5-18-1-0.1 | 38 |

In addition, the comparison of stability of Examples 13 to 16 having different compositions according to the preparation method of forming siRNA/dioTETA nanoparticles in an aqueous phase and entrapping them into polymeric micelles in the emulsion are shown in Table 14 below.

**[Table 14]**

| | Composition | ratio | siRNA dissociation(%) |
|---|---|---|---|
| Comparative Example2 | siRNA/dioTETA/mPEG-PLA-tocopherol(2k-1.7k)/DOPE | 8-18-1-1 | 46 |
| Example 13 | | 5-18-1-0.5 | 22 |
| Example 14 | | 5-18-1-1 | 12 |
| Comparative Example3 | siRNA/dioTETA/mPEG-PLA-tocopherol(2k-1.7k)/PLANa (1.7K)/DOPE | 5-18-1-0.3-1 | 32 |
| Example 15 | | 5-18-1-0.1-1 | 9 |
| Example 16 | | 5-18-1-0.3-1 | 8 |

Tables 13 and 14 show the comparison results of stability of the polymeric micelle delivery system through heparin competition. It can be seen that the delivery system prepared by an embodiment of the preparation method according to the present invention in which the preparation or the formation of siRNA/dioTETA nanoparticles was carried out in an aqueous phase, followed by entrapping into polymeric micelles in the emulsion showed low dissociation by heparin. These results demonstrate that siRNA can be stably entrapped into the polymeric micelles, thereby maintaining the stability in blood or in the body.

### [Experimental Example 4] Comparison of reproducibility of polymeric micelles according to the preparation method

The specific composition ratio and preparation method were selected to prepare formulations into 200 µg scale based on siRNA. The preparation reproducibility of the formulations was compared based on the siRNA content (yield) by repeating the same experiment three times.

The preparation reproducibility according to the preparation method of Comparative Example 1 was compared with those of Examples 3 and 7, and the results are shown in Table 15 below.

**[Table 15]**

| | Primary amount (%) | Secondary amount (%) | Tertiary amount (%) | CV (%) |
|---|---|---|---|---|
| Comparative Example1 | 25 | 42 | 10 | 16 |
| Example 3 | 55 | 63 | 58 | 4 |
| Example 7 | 70 | 75 | 79 | 4 |

| | | | | |
|---|---|---|---|---|
| (CV: Coefficient of Variation) | | | | |

### [Experimental Example 5] Comparison of siRNA content (yield) with increasing amount of polymeric micelle prepared according to the preparation method

The specific composition ratio and preparation method were selected to prepare formulations into 200 µg, 500 µg, 1000 µg scales based on siRNA. The yields according to the increase in the preparation amount were compared by repeating the same experiment two times.

The yield according to the increase in the preparation amount of Comparative Example 1 was compared with that of Example 7, and the results are shown in Table 16 below.

**[Table 16]**

| | Amount of 200 µg scale(%) | Amount of 500 µg scale(%) | Amount of 1000 µg scale(%) |
|---|---|---|---|
| Comparative Example1 | 25 | 12 | 5 |
| Example 7 | 78 | 81 | 85 |

### [Experimental Example 6] Analysis of plasma concentration of polymer micelles

The prepared formulations were administered to animals and blood was collected 0.5 hours and 6 hours after the administration, and the blood concentration of the micelles was analyzed by the following procedures using RT (Reverse Transcription) and qRT-PCR (quantitative Reverse Transcription-Polymerase Chain Reaction).

The formulations were intravenously injected into Balb/c mice at a concentration of 1 mg/kg, and blood was collected after 0.5 hour and 6 hours. The blood was centrifuged at 13000 rpm at 4°C for 15 minutes to collect only the upper layer into a new tube, and the concentration of the standard formulation was prepared by diluting with PBS to a total of 11 concentrations ranging from 4 µM to 0.00256 µM. 1 µℓ of the diluted standard formulation was added to a 96-well plate for PCR, and 9 µℓ of Balb/c mouse serum and 90 µℓ of 0.25% triton X-100 were added thereto. After adding 90 µℓ of 0.25% triton X-100 to 10 µℓ of the blood sample of the experimental group, a pretreatment step of deformulating the delivery system was carried out. The exposed siRNA as the formulation was deformulated was synthesized into cDNA through a reverse transcription (RT) step, and qRT-PCR (Bio-Rad CFX96 Real-Time System) was performed using the synthesized cDNA. The analysis was performed using the Bio-Rad CFX Manager program.

**[Table 17]**

| | Blood concentration (ng/mL) | |
|---|---|---|
| | 0.5hr | 6hr |
| Comparative Example1 | 11650 | 5363 |
| Example 3 | 14362 | 7701 |
| Example 7 | 17410 | 8042 |
| Example 14 | 12033 | 6462 |
| Example 16 | 13250 | 8634 |

## Claims

1. A̅ method for preparing a composition for delivering an anionic drug,
wherein said method (i) comprises:
(a) dissolving an anionic drug and a cationic lipid in an aqueous solvent respectively and mixing them; and
(b) dissolving an amphiphilic block copolymer in an aqueous solvent and mixing with the mixture obtained in step (a), wherein the cationic lipid is a cationic lipid represented by Chemical Formula 1: 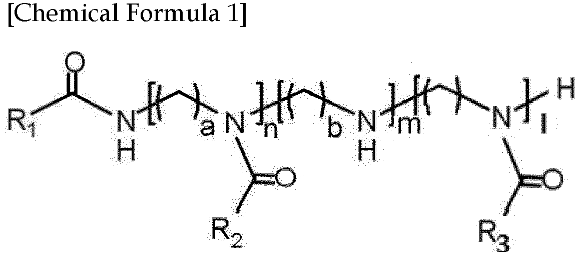 in the formula 1,
n, m and 1 are each 0 to 12, with a proviso that 1 ≤ n + m + 1 ≤ 12,
a, b and c are independently 1 to 6,
R₁, R₂ and R₃ are independently hydrogen or a saturated and unsaturated hydrocarbon having 11 to 25 carbon atoms, with a proviso that at least one of R₁, R₂ and R₃ is a saturated or unsaturated hydrocarbon having 11 to 25 carbon atoms, and
wherein the amphiphilic block copolymer is an A-B type block copolymer comprising a hydrophilic block (A) and a hydrophobic block (B) where the hydrophilic A block is polyethylene glycol or monomethoxypolyethyleneglycol, and the hydrophobic B block is polylactide, polygylcolide or coplolymer thereof; and
wherein the steps (a) and (b) are conducted in a monophase, or
said method (ii) comprises:
(a') dissolving an anionic drug and the cationic lipid represented by Chemical Formula 1 as defined above in an aqueous solvent respectively and mixing them, followed by freeze-drying;
(b-1) dissolving the freeze-dried product obtained in step (a') in an organic solvent;
(b-2) mixing the solution obtained in step (b-1) with an aqueous solvent; and
(b-3) removing the organic solvent from the mixture obtained in step (b-2), wherein the amphiphilic block copolymer is dissolved in the organic solvent of step (b-1) or the aqueous solvent of step (b-2).

2. The method of claim 1, comprising further dissolving a polylactic acid salt in an aqueous solvent or an organic solvent and mixing with the mixture obtained in step (a), in step (b).

3. The method of claim 1, further comprising (c) of stabilizing the mixture obtained in step (b) at a temperature of 0°C to 50°C for 5 minutes to 60 minutes.

4. The method of any one of claims 1 to 3, wherein the volume ratio (aqueous solution of cationic lipid/aqueous solution of anionic drug) of the aqueous solution in which the cationic lipid is dissolved to the aqueous solution in which the anionic drug is dissolved in step (a) or step (a') is 1 to 20.

5. The method of any one of claims 1 to 3, wherein the anionic drug is a peptide, a protein or a nucleic acid.

6. The method of claim 6, wherein at least one terminal of the nucleic acid is modified with at least one selected from the group consisting of cholesterol, tocopherol, and a fatty acid having 10 to 24 carbon atoms.

7. The method of any one of claims 1 to 3, wherein the organic solvent in step (b) or (b-1) is at least one selected from the group consisting of acetone, ethanol, methanol, methylene chloride, chloroform, dioxane, dimethyl sulfoxide, acetonitrile, ethyl acetate and acetic acid.

8. The method of claim 2, wherein the polylactic acid salt is at least one selected from the group consisting of the compounds represented by Chemical Formulae 2 to 7:
[Chemical Formula 2] RO-CHZ-[A]ₙ-[B]ₘ-COOM
in the formula 2, A is -COO-CHZ-; B is -COO-CHY-, -COO-CH₂CH₂CH₂CH₂CH₂- or -COO-CH₂CH₂OCH₂; R is a hydrogen, an acetyl, benzoyl, decanoyl, palmitoyl, methyl, or ethyl group; Z and Y are independently a hydrogen, a methyl or phenyl group; M is Na, K, or Li; n is an integer of 1 to 30; and m is an integer of 0 to 20;
[Chemical Formula 3] RO-CHZ-[COO-CHX]ₚ-[COO-CHY' ]_{q}-COO-CHZ-COOM
in the formula 3, X is a methyl group; Y' is a hydrogen or a phenyl group; p is an integer of 0 to 25 and q is an integer of 0 to 25, with a proviso that p+q is an integer of 5 to 25; R is a hydrogen, an acetyl, benzoyl, decanoyl, palmitoyl, methyl or ethyl group; M is Na, K, or Li; Z is a hydrogen, a methyl or phenyl group;
[Chemical Formula 4] RO-PAD-COO-W-M'
in the formula 4, W-M' is 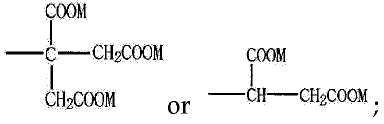 PAD is selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; R is a hydrogen, a acetyl, benzoyl, decanoyl, palmitoyl, methyl or ethyl group; M is independently Na, K, or Li;
[Chemical Formula 5] S-O-PAD-COO-Q
in the formula 5, S is 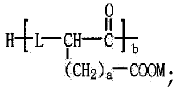 L is -NR₁- or -0-, herein R₁ is a hydrogen or C₁₋₁₀ alkyl; Q is CH₃, CH₂CH₃, CH₂CH₂CH₃, CH₂CH₂CH₂CH₃, or CH₂C₆H₅; a is an integer of 0 to 4; b is an integer of 1 to 10; M is Na, K, or Li; PAD is at least one selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; 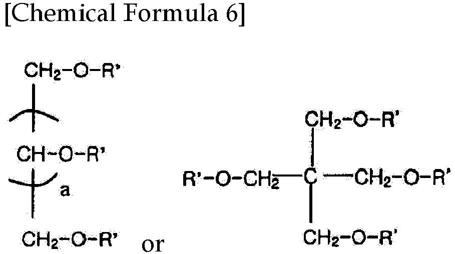 in the formula 6, R' is -PAD-O-C(O)-CH₂CH₂-C(O)-OM, PAD is selected from the group consisting of D,L-polylactic acid, D-polylactic acid, polymandelic acid, a copolymer of D,L-lactic acid and glycolic acid, a copolymer of D,L-lactic acid and mandelic acid, a copolymer of D,L-lactic acid and caprolactone, and a copolymer of D,L-lactic acid and 1,4-dioxan-2-one; M is Na, K, or Li; and a is an integer of 1 to 4;
[Chemical Formula 7] YO-[-C(O)-(CHX)ₐ-O-]ₘ-C(O)-R-C(O)-[-O-(CHX' )_{b}-C(O)-]ₙ-OZ
in the formula 7, X and X' are independently hydrogen, alkyl having 1 to 10 carbon atoms, or aryl having 6 to 20 carbon atoms; Y and Z are independently Na, K, or Li; m and n are independently integers of 0 to 95, with a proviso that 5 < m + n < 100; a and b are independently integers of 1 to 6; R is -(CH₂)ₖ-, a divalent alkenyl having 2 to 10 carbon atoms, a divalent aryl having 6 to 20 carbon atoms, or a combination thereof, wherein k is an integer of 0 to 10.

9. The method of claim 1, wherein the terminal hydroxyl group of the hydrophobic B block is modified with at least one selected from the group consisting of cholesterol, tocopherol, and a fatty acid having 10 to 24 carbon atoms.

10. The method of any one of claims 1 to 3, wherein the organic solvent comprises a fusogenic lipid.

11. The method of claim 10, wherein the fusogenic lipid is at least one selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 11,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl
phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

## Patentansprüche

1. Verfahren zur Herstellung einer Zusammensetzung zur Verabreichung eines anionischen Arzneimittels,
wobei das Verfahren (i) umfasst:
(a) Lösen eines anionischen Arzneimittels und eines kationischen Lipids in einem wässrigen Lösungsmittel und Mischen derselben; und
(b) Lösen eines amphiphilen Blockcopolymers in einem wässrigen Lösungsmittel und Mischen mit der in Schritt (a) erhaltenen Mischung, wobei das kationische Lipid ein kationisches Lipid der chemischen Formel 1 ist: 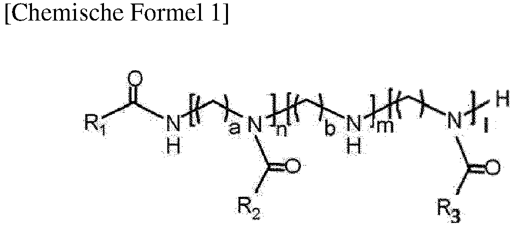 in der Formel 1,
n, m und 1 jeweils 0 bis 12 sind, mit der Maßgabe, dass 1 ≤ n + m + 1 ≤ 12 ist,
a, b und c sind unabhängig voneinander 1 bis 6,
R₁, R₂ und R₃ unabhängig voneinander Wasserstoff oder ein gesättigter und ungesättigter Kohlenwasserstoff mit 11 bis 25 Kohlenstoffatomen sind, mit der Maßgabe, dass mindestens einer der Reste R₁, R₂ und R₃ ein gesättigter oder ungesättigter Kohlenwasserstoff mit 11 bis 25 Kohlenstoffatomen ist, und
wobei das amphiphile Blockcopolymer ein Blockcopolymer vom A-B-Typ ist, das einen hydrophilen Block (A) und einen hydrophoben Block (B) umfasst, wobei der hydrophile A-Block Polyethylenglykol oder Monomethoxypolyethylenglykol ist und der hydrophobe B-Block Polylactid, Polygylcolid oder ein Copolymer davon ist; und wobei die Schritte (a) und (b) in einer Monophase durchgeführt werden, oder das Verfahren (ii) umfasst:
(a') Lösen eines anionischen Arzneimittels und des kationischen Lipids, dargestellt durch die chemische Formel 1, wie oben definiert, in einem wässrigen Lösungsmittel und Mischen derselben, gefolgt von Gefriertrocknen;
(b-1) Lösen des in Schritt (a') erhaltenen gefriergetrockneten Produkts in einem organischen Lösungsmittel;
(b-2) Mischen der in Schritt (b-1) erhaltenen Lösung mit einem wässrigen Lösungsmittel; und
(b-3) Entfernen des organischen Lösungsmittels aus der in Schritt (b-2) erhaltenen Mischung, wobei das amphiphile Blockcopolymer in dem organischen Lösungsmittel von Schritt (b-1) oder dem wässrigen Lösungsmittel von Schritt (b-2) gelöst wird.

2. Verfahren nach Anspruch 1, umfassend ferner das Lösen eines Polymilchsäuresalzes in einem wässrigen Lösungsmittel oder einem organischen Lösungsmittel und Mischen mit der in Schritt (a) erhaltenen Mischung in Schritt (b).

3. Verfahren nach Anspruch 1, umfassend ferner (c) die Stabilisierung der in Schritt (b) erhaltenen Mischung bei einer Temperatur von 0°C bis 50°C für 5 Minuten bis 60 Minuten.

4. Verfahren nach einem der Ansprüche 1 bis 3, wobei das Volumenverhältnis (wässrige Lösung des kationischen Lipids/ wässrige Lösung des anionischen Arzneimittels) der wässrigen Lösung, in der das kationische Lipid gelöst ist, zu der wässrigen Lösung, in der das anionische Arzneimittel in Schritt (a) oder Schritt (a') gelöst ist, 1 bis 20 beträgt.

5. Verfahren nach einem der Ansprüche 1 bis 3, wobei das anionische Arzneimittel ein Peptid, ein Protein oder eine Nukleinsäure ist.

6. Verfahren nach Anspruch 6, wobei mindestens ein Ende der Nukleinsäure mit mindestens einem aus der Gruppe bestehend aus Cholesterin, Tocopherol und einer Fettsäure mit 10 bis 24 Kohlenstoffatomen modifiziert ist.

7. Verfahren nach einem der Ansprüche 1 bis 3, wobei das organische Lösungsmittel in Schritt (b) oder (b-1) mindestens eines ist, ausgewählt aus der Gruppe bestehend aus Aceton, Ethanol, Methanol, Methylenchlorid, Chloroform, Dioxan, Dimethylsulfoxid, Acetonitril, Ethylacetat und Essigsäure.

8. Verfahren nach Anspruch 2, wobei das Polymilchsäuresalz mindestens eine ist, die aus der Gruppe ausgewählt ist, die aus den durch die chemischen Formeln 2 bis 7 dargestellten Verbindungen besteht:
[Chemische Formel 2] RO-CHZ-[A]ₙ-[B]ₘ-COOM
wobei in der Formel 2 A für -COO-CHZ- steht; B für -COO-CHY-, -COO-CH₂CH₂CH₂CH₂CH₂- oder -COO-CH₂CH₂OCH₂ steht; R für ein Wasserstoffatom, eine Acetyl-, Benzoyl-, Decanoyl-, Palmitoyl-, Methyl- oder Ethylgruppe steht; Z und Y unabhängig voneinander für ein Wasserstoffatom, eine Methyl- oder Phenylgruppe stehen; M für Na, K oder Li steht; n eine ganze Zahl von 1 bis 30 ist; und m eine ganze Zahl von 0 bis 20 ist;
[Chemische Formel 3] RO-CHZ-[COO-CHX]ₚ-[COO-CHY' ]_{q}-COO-CHZ-COOM
in der Formel 3 ist X eine Methylgruppe; Y' ist ein Wasserstoff oder eine Phenylgruppe; p ist eine ganze Zahl von 0 bis 25 und q ist eine ganze Zahl von 0 bis 25, mit der Maßgabe, dass p+q eine ganze Zahl von 5 bis 25 ist; R ist ein Wasserstoff, eine Acetyl-, Benzoyl-, Decanoyl-, Palmitoyl-, Methyl- oder Ethylgruppe; M ist Na, K oder Li; Z ist ein Wasserstoff, eine Methyl- oder Phenylgruppe;
[Chemische Formel 4] RO-PAD-COO-W-M'
in der Formel 4 ist W-M' 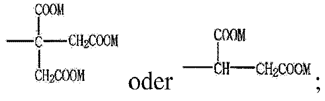 PAD ist ausgewählt aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on; R ist ein Wasserstoff, eine Acetyl-, Benzoyl-, Decanoyl-, Palmitoyl-, Methyl- oder Ethylgruppe; M ist unabhängig Na, K oder Li;
[Chemische Formel 5] S-O-PAD-COO-Q
in der Formel 5 ist S 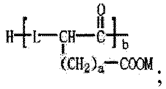 L ist -NR₁- oder -0-, wobei R₁ ein Wasserstoff oder C₁₋₁₀-Alkyl ist; Q ist CH₃, CH₂CH₃, CH₂CH₂CH₃, CH₂CH₂CH₂CH₃ oder CH2C6H5; a ist eine ganze Zahl von 0 bis 4; b ist eine ganze Zahl von 1 bis 10; M ist Na, K oder Li; PAD mindestens eines ist, ausgewählt aus der Gruppe bestehend aus D,L-Polymilchsäure, D-Polymilchsäure, Polymandelsäure, einem Copolymer aus D,L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on; 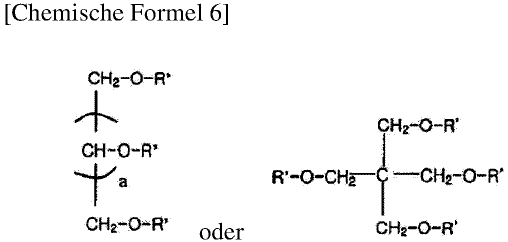 in der Formel 6 ist R' -PAD-O-C(O)-CH₂CH₂-C(O)-OM, PAD ist ausgewählt aus der Gruppe bestehend aus D,L-Polymilchsäure, D-Polymilchsäure, Polymandelsäure, einem Copolymer aus D, L-Milchsäure und Glykolsäure, einem Copolymer aus D,L-Milchsäure und Mandelsäure, einem Copolymer aus D,L-Milchsäure und Caprolacton und einem Copolymer aus D,L-Milchsäure und 1,4-Dioxan-2-on; M ist Na, K oder Li; und a ist eine ganze Zahl von 1 bis 4;
[Chemische Formel 7] YO-[-C(O)-(CHX)ₐ-O-]ₘ-C(O)-R-C(O)-[-O-(CHX' )_{b}-C(O)-]ₙ-OZ
in der Formel 7 sind X und X' unabhängig voneinander Wasserstoff, Alkyl mit 1 bis 10 Kohlenstoffatomen oder Aryl mit 6 bis 20 Kohlenstoffatomen; Y und Z sind unabhängig voneinander Na, K oder Li; m und n sind unabhängig voneinander ganze Zahlen von 0 bis 95, mit der Maßgabe, dass 5 < m + n < 100; a und b unabhängig voneinander ganze Zahlen von 1 bis 6 sind; R -(CH₂)ₖ-, ein zweiwertiges Alkenyl mit 2 bis 10 Kohlenstoffatomen, ein zweiwertiges Aryl mit 6 bis 20 Kohlenstoffatomen oder eine Kombination davon ist, wobei k eine ganze Zahl von 0 bis 10 ist.

9. Verfahren nach Anspruch 1, wobei die endständige Hydroxylgruppe des hydrophoben B-Blocks mit mindestens einem aus der Gruppe bestehend aus Cholesterin, Tocopherol und einer Fettsäure mit 10 bis 24 Kohlenstoffatomen modifiziert ist.

10. Verfahren nach einem der Ansprüche 1 bis 3, wobei das organische Lösungsmittel ein fusogenes Lipid umfasst.

11. Verfahren nach Anspruch 10, wobei das fusogene Lipid mindestens eines ist, ausgewählt aus der Gruppe bestehend aus Dilauroylphosphatidylethanolamin, Dimyristoylphosphatidylethanolamin, Dipalmitoylphosphatidylethanolamin, Distearoylphosphatidylethanolamin, Dioleoylphosphatidylethanolamin, Dilinoleoylphosphatidylethanolamin, 1-Palmitoyl-2-olcoylphosphatidylethanolamin, 11,2-Diphytanoyl-3-sn-phosphatidylethanolamin, Dilauroylphosphatidylcholin, Dimyristoylphosphatidylcholin, Dipalmitoylphosphatidylcholin, Distearoylphosphatidylcholin, Dioleoylphosphatidylcholin, Dilinoleoylphosphatidylcholin, 1-Palmitoyl-2-Oleoyl-Phosphatidylcholin, 1,2-Diphytanoyl-3-sn-Phosphatidylcholin, Dilauroyl-Phosphatidsäure, Dimyristoylphosphatidsäure, Dipalmitoylphosphatidsäure, Distearoylphosphatidsäure, Dioleoylphosphatidsäure, Dilinoleoylphosphatidsäure, 1-Palmitoyl-2-oleoylphosphatidsäure, 1,2-Diphytanoyl-3-sn-phosphatidsäure, Cholesterin und Tocopherol.

## Revendications

1. Procédé de préparation d'une composition pour délivrer un médicament anionique,
dans lequel ledit procédé (i) comprend :
(a) la dissolution d'un médicament anionique et d'un lipide cationique respectivement dans un solvant aqueux et leur mélange ; et
(b) la dissolution d'un copolymère séquencé amphiphile dans un solvant aqueux et le mélange avec le mélange obtenu dans l'étape (a), dans lequel le lipide cationique est un lipide cationique représenté par la formule chimique 1 : 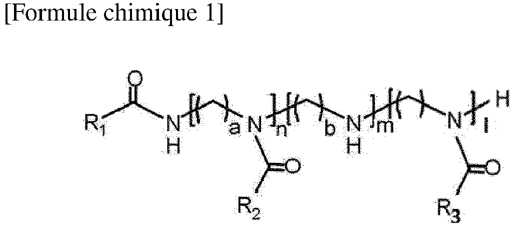 dans la formule 1,
n, m et l sont chacun 0 à 12, à condition que 1 ≤ n + m + l ≤ 12,
a, b et c sont indépendamment de 1 à 6,
R₁, R₂ et R₃ sont indépendamment de l'hydrogène ou un hydrocarbure saturé et insaturé ayant 11 à 25 atomes de carbone, à condition qu'au moins un des R₁, R₂ et R₃ soit un hydrocarbure saturé ou insaturé ayant 11 à 25 atomes de carbone, et
dans lequel le copolymère séquencé amphiphile est un copolymère séquencé de type A-B comprenant un bloc hydrophile (A) et un bloc hydrophobe (B) où le bloc hydrophile A est un polyéthylène glycol ou un monométhoxypolyéthylèneglycol, et le bloc hydrophobe B est un polylactide, un polygylcolide ou un copolymère de ceux-ci ; et
dans lequel les étapes (a) et (b) sont réalisées dans une monophase, ou ledit procédé (ii) comprend :
(a') la dissolution d'un médicament anionique et du lipide cationique représenté par la formule chimique 1 telle que définie ci-dessus dans un solvant aqueux respectivement et leur mélange, suivi d'une lyophilisation ;
(b-1) dissoudre le produit lyophilisé obtenu dans l'étape (a') dans un solvant organique ;
(b-2) mélanger la solution obtenue à l'étape (b-1) avec un solvant aqueux ; et
(b-3) éliminer le solvant organique du mélange obtenu dans l'étape (b-2), dans lequel le copolymère séquencé amphiphile est dissous dans le solvant organique de l'étape (b-1) ou le solvant aqueux de l'étape (b-2).

2. Procédé de la revendication 1, comprenant en outre la dissolution d'un sel d'acide polylactique dans un solvant aqueux ou un solvant organique et le mélange avec le mélange obtenu dans l'étape (a), dans l'étape (b).

3. Procédé de la revendication 1, comprenant en outre (c) la stabilisation du mélange obtenu à l'étape (b) à une température de 0°C à 50°C pendant 5 minutes à 60 minutes.

4. Procédé de l'une quelconque des revendications 1 à 3, dans lequel le rapport volumique (solution aqueuse de lipide cationique/solution aqueuse de médicament anionique) de la solution aqueuse dans laquelle le lipide cationique est dissous à la solution aqueuse dans laquelle le médicament anionique est dissous dans l'étape (a) ou l'étape (a') est de 1 à 20.

5. Procédé de l'une quelconque des revendications 1 à 3, dans lequel le médicament anionique est un peptide, une protéine ou un acide nucléique.

6. Procédé de la revendication 6, dans lequel au moins une extrémité de l'acide nucléique est modifiée avec au moins un élément choisi dans le groupe constitué par le cholestérol, le tocophérol et un acide gras ayant 10 à 24 atomes de carbone.

7. Procédé de l'une quelconque des revendications 1 à 3, dans lequel le solvant organique de l'étape (b) ou (b-1) est au moins un choisi dans le groupe constitué par l'acétone, l'éthanol, le méthanol, le chlorure de méthylène, le chloroforme, le dioxane, le diméthylsulfoxyde, l'acétonitrile, l'acétate d'éthyle et l'acide acétique.

8. Procédé de la revendication 2, dans lequel le sel d'acide polylactique est au moins un choisi dans le groupe constitué par les composés représentés par les formules chimiques 2 à 7 :
[Formule chimique 2] RO―CHZ―[A]ₙ―[B]ₘ―COOM
dans la formule 2, A est -COO-CHZ- ; B est -COO-CHY-, -COO-CH₂CH₂CH₂CH₂CH₂- ou -COO-CH₂CH₂OCH₂ ; R est un hydrogène, un groupe acétyle, benzoyle, décanoyle, palmitoyle, méthyle ou éthyle ; Z et Y sont indépendamment un hydrogène, un groupe méthyle ou phényle ; M est Na, K ou Li ; n est un nombre entier de 1 à 30 ; et m est un nombre entier de 0 à 20 ;
[Formule chimique 3] RO―CHZ―[COO-CHX]ₚ―[COO-CHY' ]_{q}―COO―CHZ―COOM
dans la formule 3, X est un groupe méthyle ; Y' est un hydrogène ou un groupe phényle ; p est un nombre entier de 0 à 25 et q est un nombre entier de 0 à 25, à condition que p+q soit un nombre entier de 5 à 25 ; R est un hydrogène, un groupe acétyle, benzoyle, décanoyle, palmitoyle, méthyle ou éthyle ; M est Na, K ou Li ; Z est un hydrogène, un groupe méthyle ou phényle ;
[Formule chimique 4] RO―PAD―COO―W―M'
dans la formule 4, W-M' est 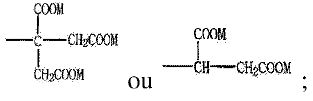 PAD est choisi dans le groupe constitué par l'acide D,L-polylactique, l'acide D-polylactique, l'acide polymandélique, un copolymère de l'acide D,L-lactique et de l'acide glycolique, un copolymère de l'acide D,L-lactique et de l'acide mandélique, un copolymère de l'acide D,L-lactique et de la caprolactone, et un copolymère de l'acide D,L-lactique et de la 1,4-dioxan-2-one ; R est un hydrogène, un groupe acétyle, benzoyle, décanoyle, palmitoyle, méthyle ou éthyle ; M est indépendamment Na, K ou Li ;
[Formule chimique 5] S―O―PAD―COO―Q
dans la formule 5, S est 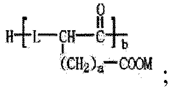 L est -NR₁- ou -0-, où R₁ est un hydrogène ou un alkyle en C₁₋₁₀ ; Q est CH3, CH2CH3, CH2CH2CH3, CH2CH2CH2CH3, ou CH₂C₆H₅ ; a est un nombre entier de 0 à 4 ; b est un nombre entier de 1 à 10 ; M est Na, K, ou Li ; PAD est au moins un élément choisi dans le groupe constitué par l'acide D,L-polylactique, l'acide D-polylactique, l'acide polymandélique, un copolymère de l'acide D,L-lactique et de l'acide glycolique, un copolymère de l'acide D,L-lactique et de l'acide mandélique, un copolymère de l'acide D,L-lactique et de la caprolactone, et un copolymère de l'acide D,L-lactique et de la l,4-dioxan-2-one ; 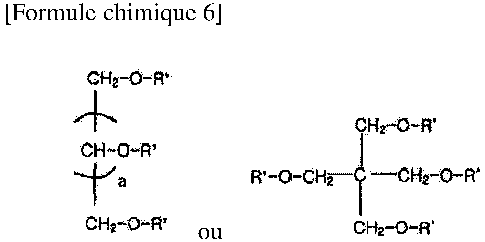 dans la formule 6, R' est -PAD-O-C(O)-CH₂CH₂-C(O)-OM, PAD est choisi dans le groupe constitué par l'acide D,L-polylactique, l'acide D-polylactique, l'acide polymandélique, un copolymère d'acide D, L-lactique et d'acide glycolique, un copolymère d'acide D,L-lactique et d'acide mandélique, un copolymère d'acide D,L-lactique et de caprolactone, et un copolymère d'acide D,L-lactique et de 1,4-dioxan-2-one ; M est Na, K ou Li ; et a est un nombre entier de 1 à 4 ;
[Formule chimique 7] YO―[―C(O)―(CHX)ₐ―O―]ₘ―C(O)―R―C(O)―[―O―(CHX' )_{b}―C(O)―]_{n―}OZ
dans la formule 7, X et X' sont indépendamment un hydrogène, un alkyle ayant 1 à 10 atomes de carbone, ou un aryle ayant 6 à 20 atomes de carbone ; Y et Z sont indépendamment Na, K ou Li ; m et n sont indépendamment des entiers de 0 à 95, à condition que 5 < m + n < 100 ; a et b sont indépendamment des entiers de 1 à 6 ; R est -(CH₂)ₖ-, un alcényle divalent ayant 2 à 10 atomes de carbone, un aryle divalent ayant 6 à 20 atomes de carbone, ou une combinaison de ceux-ci, où k est un entier de 0 à 10.

9. Procédé de la revendication 1, dans lequel le groupe hydroxyle terminal du bloc B hydrophobe est modifié avec au moins un élément choisi dans le groupe constitué par le cholestérol, le tocophérol et un acide gras ayant 10 à 24 atomes de carbone.

10. Procédé de l'une quelconque des revendications 1 à 3, dans lequel le solvant organique comprend un lipide fusogène.

11. Procédé de la revendication 10, dans lequel le lipide fusogène est au moins un choisi dans le groupe constitué par la dilauroyl phosphatidyléthanolamine, la dimyristoyl phosphatidyléthanolamine, la dipalmitoyl phosphatidyléthanolamine, la distéaroyl phosphatidyléthanolamine, la dioléoyl phosphatidyléthanolamine, dilinoléoyl phosphatidyléthanolamine, 1-palmitoyl-2-oléoyl phosphatidyléthanolamine, 11,2-diphytanoyl-3-sn-phosphatidyléthanolamine, dilauroyl phosphatidylcholine, dimyristoyl phosphatidylcholine, dipalmitoyl phosphatidylcholine, distéaroyl phosphatidylcholine, dioléoyl phosphatidylcholine, dilinoléoyl phosphatidylcholine, 1-palmitoyl-2-oléoyl phosphatidylcholine, 1,2-diphytanoyl-3-sn-phosphatidylcholine, l'acide dilauroyl phosphatidique, l'acide dimyristoyl phosphatidique, l'acide dipalmitoyl phosphatidique, l'acide distearoyl phosphatidique, l'acide dioléoyl phosphatidique, l'acide dilinoléoyl phosphatidique, l'acide 1-palmitoyl-2-oléoyl phosphatidique, l'acide 1,2-diphytanoyl-3-sn-phosphatidique, le cholestérol et le tocophérol.
